# EUROPEAN PATENT APPLICATION

(11) **EP 4 691 441 A1**
(43) Date of publication of application: **11.02.2026**
(21) Application number: 24792698.3
(22) Date of filing: 17.04.2024
(51) Int. Cl.: A61F 13/15, A61F 13/49

(54) **WEARABLE ARTICLE MANUFACTURING METHOD**

(30) Priority: 17.04.2023 JP 2023067283
(71) Applicant: Zuiko Corporation, Ibaraki-shi Osaka 5670082 (JP)
(72) Inventor: UMEBAYASHI, Toyoshi, Ibaraki-shi, Osaka 567-0082 (JP)
(74) Representative: SSM Sandmair
(86) International application number: PCT/JP2024/015279
(87) International publication number: WO 2024/219426

(57) **Abstract**

Provided is a wearable article manufacturing method that can downsize manufacturing equipment. The wearable article manufacturing method is a method of manufacturing a wearable article having an absorbent body that is disposed in the crotch of a wearer and extends in a first direction between a first end and a second end, a first waist member that is connected to the absorbent body on the first end side and is disposed on the front waist of the wearer when the wearable article is worn, and a second waist member that is connected to the absorbent body on the second end side and is disposed on the back waist of the wearer when the wearable article is worn. The wearable article manufacturing method includes a process of conveying in a second direction (D2) a sheet (S) that is to be formed into the first waist member and the second waist member and a folding process. The second direction is a direction orthogonal to the width direction (W) of the sheet, and the width direction of the sheet is a direction along the waist of the wearer when the wearable article is worn by the wearer. In the folding process, both ends in the width direction of the conveyed sheet are folded toward a center of the sheet in the width direction.

## Description

### TECHNICAL FIELD

This invention relates to a manufacturing method of a wearable article.

### BACKGROUND ART

A method of manufacturing a wearable article having an absorbent body and a pair of waist members such as described in Patent Document 1 (JP-A No. 2021-171299) is known.

In the wearable article manufacturing method of Patent Document 1 (JP-A No. 2021-171299), a first sheet for forming one of the waist members and a second sheet for forming the other of the waist members are conveyed parallel to each other with a gap between them (with a space for attaching the absorbent body), and here the absorbent body is attached so as to straddle the first sheet and the second sheet.

### CITATION LIST

### Patent Literature

Patent Document 1: JP-A No. 2021-171299

### SUMMARY OF INVENTION

### Technical Problem

In the wearable article manufacturing method disclosed in Patent Document 1 (JP-A No. 2021-171299), since the first sheet and the second sheet are conveyed parallel to each other with a gap between them, the width of manufacturing equipment in the direction orthogonal to the conveyance direction of the first sheet and the second sheet tends to increase.

### Solution to Problem

A method of manufacturing a wearable article of an embodiment of this invention is a method of manufacturing a wearable article having an absorbent body, a first waist member, and a second waist member. The absorbent body is disposed in the crotch of a wearer and extends in a first direction between a first end and a second end. The first waist member is connected to the absorbent body on the first end side and is disposed on the front waist of the wearer when the wearable article is worn. The second waist member is connected to the absorbent body on the second end side and is disposed on the back waist of the wearer when the wearable article is worn. The method of manufacturing the wearable article includes a process of conveying in a second direction a sheet that is to be formed into the first waist member and the second waist member and a folding process. The second direction is a direction orthogonal to the width direction of the sheet, and the width direction of the sheet is a direction along the waist of the wearer when the wearable article is worn by the wearer. In the folding process, both ends in the width direction of the conveyed sheet are folded toward a center side of the sheet in the width direction.

### Advantageous Effects of Invention

In the method of manufacturing the wearable article of this invention, rather than a first sheet for forming the first waist member and a second sheet for forming the second waist member being conveyed parallel to each other with a gap between them, the sheet for forming the first waist member and the second waist member is conveyed in a direction orthogonal to the width direction (the direction around the waist when the wearable article is worn) and is further conveyed after being folded in the width direction. For that reason, in the wearable article manufacturing method of the first aspect, at least manufacturing equipment downstream of the sheet folding process can be downsized.

### BRIEF DESCRIPTION OF DRAWINGS

FIG. 1 is a drawing schematically illustrating a diaper, which is an example of a wearable article manufactured using the wearable article manufacturing method of the invention of this application, in an unfolded state and shows a non-skin surface side of the diaper.
FIG. 2 is a drawing showing a skin surface side of the diaper of FIG. 1.
FIG. 3 is process diagram showing some processes in the wearable article manufacturing method pertaining to an embodiment of this invention.
FIG. 4 is a process diagram showing some processes in the wearable article manufacturing method pertaining to the embodiment of this invention and is a continuation of the processes shown in FIG. 3.
FIG. 5 is a flowchart of the wearable article manufacturing method pertaining to the embodiment of this invention.
FIG. 6 is a drawing showing the diaper of FIG. 1 as viewed from the skin surface side and schematically shows places where material is reduced in the diaper of FIG. 1.
FIG. 7 is a drawing showing a conventional diaper as viewed from the skin surface side and schematically shows the usage of material in the conventional diaper.

### DESCRIPTION OF EMBODIMENT

An embodiment of a method of manufacturing a wearable article pertaining to this invention will be described below with reference to the drawings.

It will be noted that the embodiment described below is merely an example of this invention and is not intended to limit the scope of this invention. It will be understood by persons skilled in the art that many changes may be made to the following embodiment without departing from the spirit and scope of this invention set forth in the claims.

### (1) Wearable Article

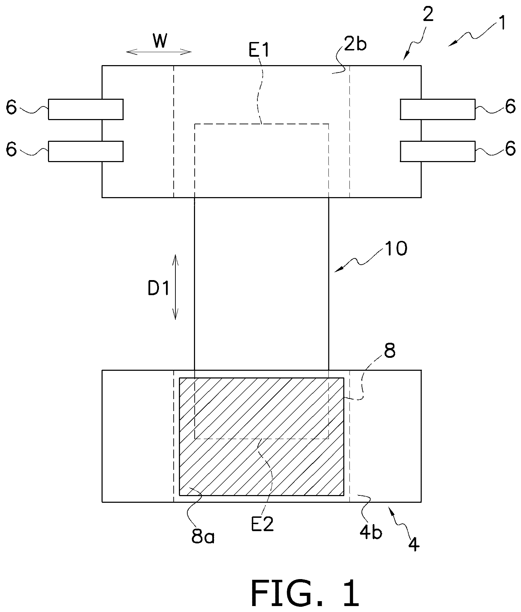

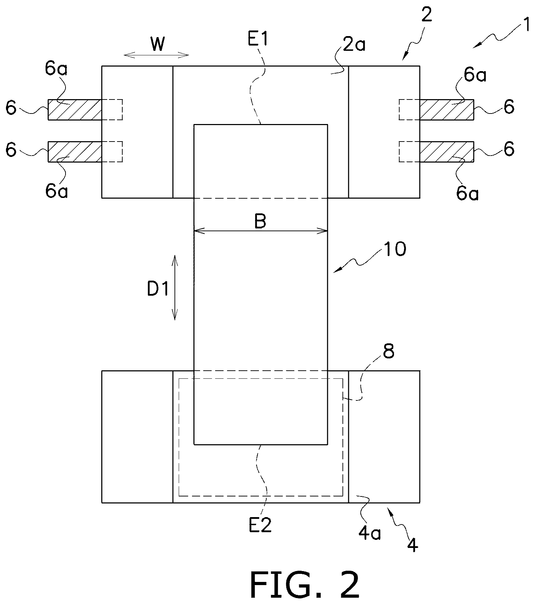

First, an example of a wearable article manufactured by a method of manufacturing wearable article of this invention will be described with reference to FIG. 1 and FIG. 2.

FIG. 1 is a drawing schematically illustrating a diaper 1, which is an example of a wearable article, in an unfolded state and shows a non-skin surface side of the diaper 1. FIG. 2 is a drawing showing a skin surface side of the diaper 1. It will be noted that here "non-skin surface" is a surface which, when a wearer wears the diaper 1, does not face the wearer's skin (is disposed on the outside). "Skin surface" is a surface which, when the wearer wears the diaper 1, faces the wearer's skin (is disposed on the inside).

In this embodiment, the wearable article will be described using as an example a type of the diaper 1 where a front waist member 2 and a back waist member 4 are secured to the wearer's waist using hook tapes 6 and a loop tape 8. However, the wearable article is not limited to the type of diaper 1 described here as long as it is a wearable article capable of utilizing the manufacturing method of this disclosure. For example, the wearable article may be a pants-type diaper manufactured utilizing the manufacturing method of this disclosure.

The diaper 1 will now be described in detail.

It will be noted that in the following description expressions such as "front," "back," "left," "right," "upper," and "lower" may be used, and unless otherwise specified these expressions represent "front," "back," "left," "right," "upper," and "lower" based on the directions seen from the perspective of the wearer when the diaper 1 is worn by the wearer.

As shown in FIG. 1, the diaper 1 has a bilaterally symmetrical structure. As shown in FIG. 1 and FIG. 2, the diaper 1 mainly has an absorbent body 10, a front waist member 2, and a back waist member 4. The front waist member 2 is an example of a first waist member in the claims. The back waist member 4 is an example of a second waist member in the claims.

As shown in FIG. 2, the absorbent body 10 is a member that extends in a first direction D1 between a first end E1 and a second end E2. The absorbent body 10 is a member that is disposed in the crotch of the wearer and covers the crotch of the wearer. The absorbent body 10 mainly includes a top sheet, a back sheet, and an absorbent core that is disposed between the top sheet and the back sheet (not shown in the drawings). The top sheet is a sheet disposed on the skin side of the wearer and is a liquid-permeable sheet. The back sheet is a sheet disposed on the non-skin side and is a liquid-impermeable sheet. The absorbent core is made of ground pulp or a mixture of ground pulp and a superabsorbent polymer. The absorbent core absorbs bodily fluids from the wearer, such as urine.

The front waist member 2 and the back waist member 4 are mainly formed from a sheet S described below. Furthermore, elastic members for fitting the diaper 1 to the wearer and sheets that are bonded to the sheet S may be disposed in appropriate places on the front waist member 2 and the back waist member 4.

The front waist member 2 is a member that is disposed on the front waist of the wearer when the diaper 1 is worn. The front waist member 2 has a skin surface 2a that faces the wearer's skin when the diaper 1 is worn and a non-skin surface 2b that is disposed opposite from the skin surface 2a and does not face the wearer's skin when the diaper 1 is worn. The front waist member 2 is connected to the absorbent body 10 on the first end E1 side of the absorbent body 10. It will be noted that preferably, as shown in FIG. 2, the first end E1 of the absorbent body 10 is disposed in a position away from the upper end portion of the front waist member 2.

As shown in FIG. 1, hook tapes 6 of a hook-and-loop fastener that are an example of tape-like members are attached to the non-skin side 2b of the front waist member 2. The hook tapes 6 have hook surfaces 6a (see the hatched portions in FIG. 2) provided with hooked naps. The hook tapes 6 are attached to the front waist member 2 so that the hook surfaces 6a are disposed on the skin surface 2a side of the front waist member 2 in a state in which the diaper 1 has been unfolded. It will be noted that although in FIG. 1 and FIG. 2 the front waist member 2 has two hook tapes 6 each attached to the left and right end portions of the front waist member 2, the number of the hook tapes 6 is merely an example. For example, just one hook tape 6 may be attached to each of the left and right end portions of the front waist member 2.

The back waist member 4 is a member that is disposed on the back waist of the wearer when the diaper 1 is worn. The back waist member 4 has a skin surface 4a that faces the wearer's skin when the diaper 1 is worn and a non-skin surface 4b that is disposed opposite from the skin surface 4a and does not face the wearer's skin when the diaper 1 is worn. The back waist member 4 is connected to the absorbent body 10 on the second end E2 side of the absorbent body 10. It will be noted that preferably, as shown in FIG. 2, the second end E2 of the absorbent body 10 is away from the upper end portion of the back waist member 4.

As shown in FIG. 1, a loop tape 8 of the hook-and-loop fastener is attached to the non-skin surface 4b of the back waist member 4. The loop tape 8 has a loop surface 8a (see the hatched portion in FIG. 1) provided with a looped nap. The loop tape 8 is attached to the back waist member 4 so that the loop surface 8a is visible when the back waist member 4 is viewed from the non-skin surface side (so that it is disposed on the non-skin surface 4b side of the back waist member 4). It will be noted that although the loop tape 8 here is a separate member from the back waist member 4 and is attached to the back waist member 4, it is not essential for the loop tape 8 to be a separate member. For example, a predetermined process may be performed on the back waist member 4 to form on the non-skin surface 4b of the back waist member 4 a loop surface on which are disposed loops with which the hooks of the hook surfaces 6a of the hook tapes 6 engage.

### (2) Wearable Article Manufacturing Method

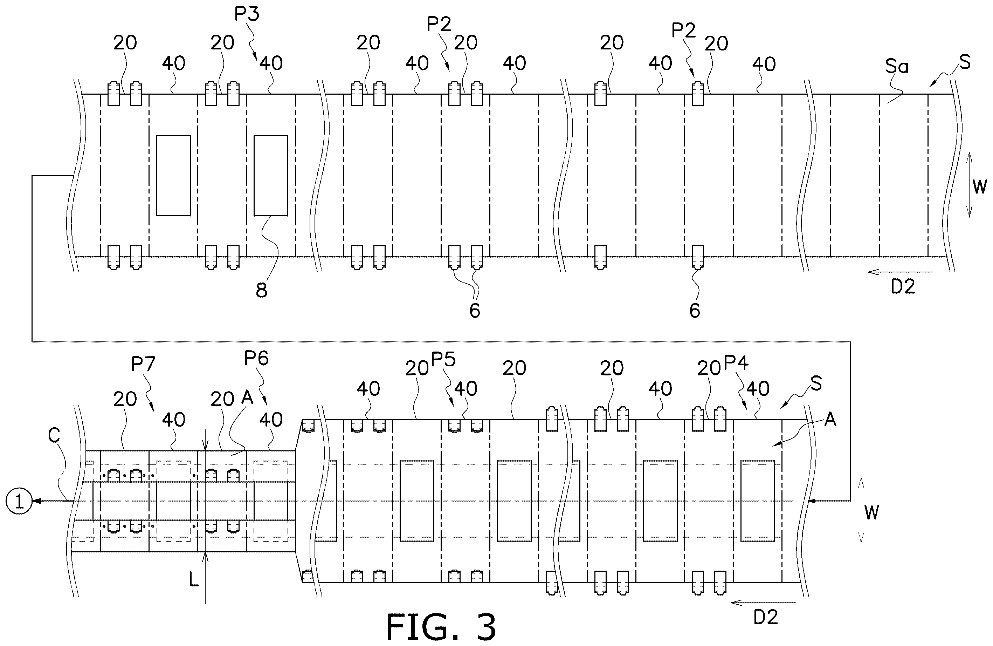

A method of manufacturing the diaper 1 will now be described with reference to FIG. 3 to FIG. 5.

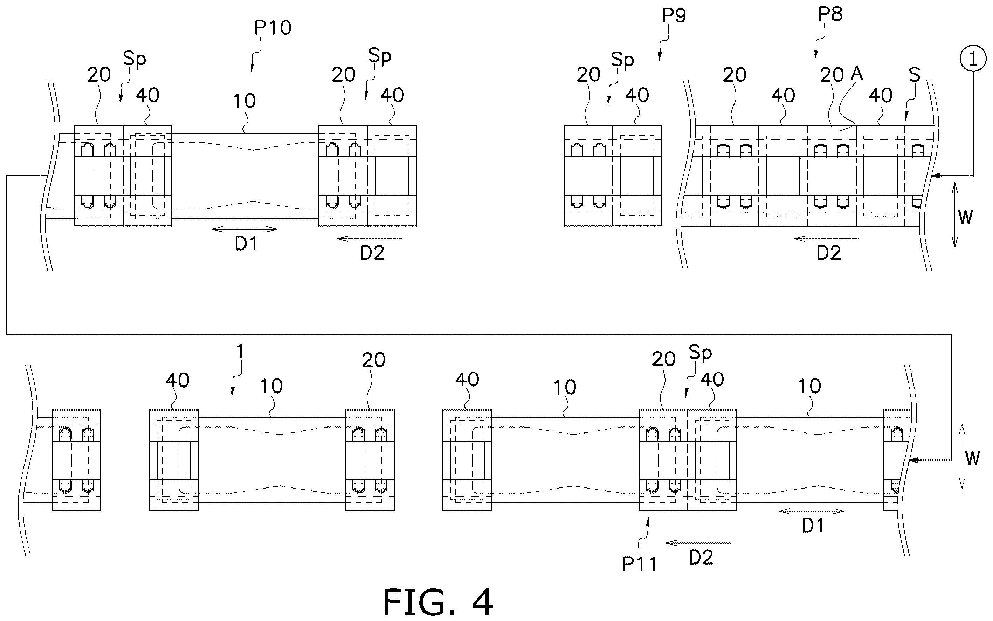

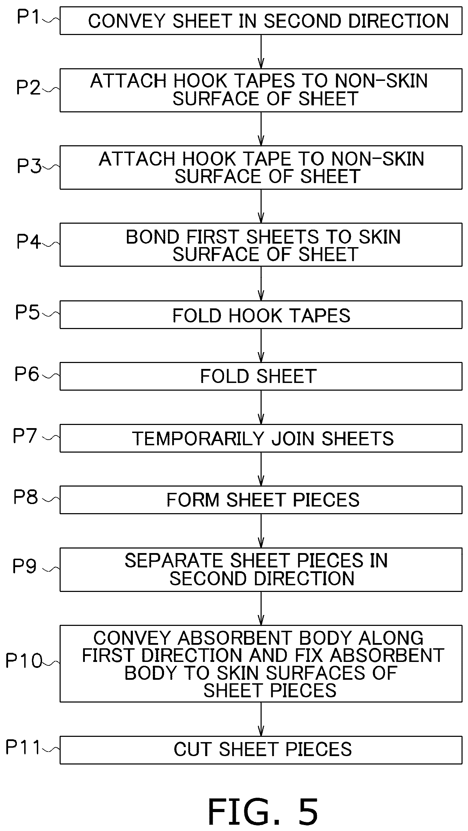

FIG. 3 is a process diagram showing some processes in the wearable article manufacturing method. FIG. 4 is a process diagram showing some processes in the wearable article manufacturing method and is a continuation of the processes shown in FIG. 3. FIG. 5 is a flowchart of the wearable article manufacturing method pertaining to the embodiment of this invention. It will be noted that the surface of the sheet S that is visible in FIG. 3 and FIG. 4 is a non-skin surface Sa that does not face the wearer's skin when the sheet S has been formed into the front waist member 2 and the back waist member 4.

In the diaper 1 manufacturing method of this disclosure, the front waist member 2 and the back waist member 4 are formed mainly from the sheet S. It will be noted that in this disclosure the expression that the front waist member 2 and the back waist member 4 are formed from the sheet S is not limited to a case where the front waist member 2 and the back waist member 4 are formed only from the sheet S. In other words, the expression that the front waist member 2 and the back waist member 4 are formed from the sheet S is not intended to exclude a case where the front waist member 2 and the back waist member 4 are manufactured using, in addition to the sheet S, members other than the sheet S (e.g., first sheets A described below).

The sheet S here is formed by layering multiple types of sheets, for example, though this is not intended to be limiting. However, the sheet S is not limited to this and may be a single-layer sheet. Description of the structure of the sheet S will be omitted here.

In the manufacturing method of the diaper 1, the sheet S is conveyed in a second direction D2 by a conveyance device (process P1). It will be noted that the second direction D2, which is the conveyance direction of the sheet S, is a direction orthogonal to a width direction W of the sheet S. The width direction W is a direction along the waist of the wearer when the sheet S has been formed into the front waist member 2 and the back waist member 4.

In the manufacturing method of the diaper 1 of this disclosure, the front waist member 2 and the back waist member 4 are alternately formed from the sheet S in the second direction D2, which is the conveyance direction of the sheet S. In FIG. 3 and FIG. 4, parts that eventually become the front waist members 2 are denoted by reference numeral "20" and parts that eventually become the back waist members 4 are denoted by reference numeral "40." It will be noted that in FIG. 3 and FIG. 4 positions where the sheet S is cut in a downstream process are illustrated with long dashed double-short dashed lines to make it easier to understand which parts eventually become the front waist members 2 and which parts eventually become the back waist members 4. It will be noted that the long dashed double-short dashed lines are for reference only and are not intended to indicate that the sheet S has already been cut at these positions.

The hook tapes 6 are attached by a hook tape joining device to both end portions, in the width direction W, of the sheet S conveyed in the second direction D2 (process P2). Specifically, the hook tape joining device disposes the hook tapes 6 on and fixes them to the non-skin surface Sa of the both end portions, in the width direction W, of the parts 20 that eventually become the front waist members 2 of the sheet S conveyed in the second direction D2. It will be noted that in process P2 the hook tapes 6 are attached to the non-skin surface Sa of the sheet S in an attitude such that the hook surfaces 6a are disposed on the side that is not visible when viewing the non-skin surface Sa of the sheet S. It will be noted that the hook tapes 6 may be fixed to the sheet S using an adhesive or may be fixed to the sheet S by a method such as welding or ultrasonic welding.

Moreover, the loop tapes 8 are attached by a loop tape joining device to the center portion, in the width direction W, of the sheet S conveyed in the second direction D2 (process P3). Specifically, the loop tape joining device disposes the loop tapes 8 on and fixes them to the non-skin surface Sa of the center portions, in the width direction W, of the parts 40 that eventually become the back waist members 4 of the sheet S conveyed in the second direction D2. It will be noted that the loop tapes 8 are attached to the non-skin surface Sa of the sheet S so that the loop surfaces 8a are disposed on the side that is visible when viewing the non-skin surface Sa of the sheet S. It will be noted that the loop tapes 8 may be fixed to the sheet S using an adhesive or may be fixed to the sheet S by a method such as welding or ultrasonic welding.

Next, a first sheet joining device bonds first sheets A, which are separate from the sheet S and serve as auxiliary sheets, by an appropriate method (e.g., adhesion, welding, or ultrasonic welding) to the surface on the reverse side of the non-skin surface Sa (the skin surface that faces the wearer's skin when the sheet S has been formed into the front waist member 2 and the back waist member 4) of each of both end portions, in the width direction W, of the sheet S conveyed in the second direction D2 (process P4). Each first sheet A is a sheet that extends continuously along the second direction D2.

Describing now a preferred aspect using FIG. 3, each of the pair of first sheets A that are bonded to the sheet S are bonded to areas of the sheet S from each of both edge portions, in the width direction W, to a dashed line extending in the second direction D2 on a side close to each edge portion. In other words, in FIG. 3, the first sheets A are not disposed in the area of sheet S between the two dashed lines extending in the second direction D2. In still other words, in the process of bonding the first sheets A, the first sheet A that is bonded to one end portion in the width direction W and the first sheet A that is bonded to the other end portion in the width direction W are disposed apart from each other in the width direction W.

It will be noted that in order to curb the amount of material, it is preferred that the first sheets A be disposed only in partial areas of the sheet S, but a single first sheet A may be disposed over the entire area of the sheet S.

Next, the hook tapes 6 provided on both end portions in the width direction W of the sheet S are folded toward the center of the sheet S in the width direction by a tape folding device (process P5). More specifically, the tape folding device folds, at the positions of the edge portions of the sheet S in the width direction W, each of the hook tapes 6 provided on both width direction W end portions of the sheet S toward the center C side, in the width direction W, of the sheet S on the side that becomes the skin surface of the sheet S.

Next, a sheet folding device folds, toward the center C side in the width direction W of the sheet S, both ends in the width direction W of the sheet S conveyed in the second direction D2 (process P6). As a result, at least part of the non-skin surface Sa of the sheet S faces another part of the non-skin surface Sa of the sheet S. Furthermore, when viewing the non-skin surface Sa side of the sheet S, at least parts of the first sheets A that have been bonded to the skin surface of the sheet S become visible. It will be noted that a length L (see FIG. 3) of the sheet S in the width direction W after the sheet S has been folded by this folding process is preferably 1.0 times to 1.3 times a width B (see FIG. 2) of the absorbent body 10 in a third direction D3 orthogonal to the first direction D1. More preferably, the length L of the sheet S in the width direction W after the sheet S has been folded by this folding process is preferably 1.05 times to 1.2 times the width B of the absorbent body 10 in the third direction D3 orthogonal to the first direction D1.

In this way, in the diaper 1 manufacturing method of this disclosure, rather than a first sheet for forming the front waist member 2 and a second sheet for forming the back waist member 4 being conveyed parallel to each other with a gap between them, the sheet S for forming the front waist member 2 and the back waist member 4 is conveyed in a direction orthogonal to the width direction W and is further conveyed after being folded in the width direction W. For that reason, in the diaper 1 manufacturing method of this disclosure, at least manufacturing equipment downstream of the sheet S folding process (process P6) can be downsized. Depending on how large the length L is relative to the width B, the width of the conveyance path of the sheet S can be reduced by about 40% (compared with a case where the sheet S is not folded) by folding the sheet S in the width direction W of the sheet S.

After the sheet S has been folded in process P6, the overlapping parts of the sheet S are temporarily fastened to each other by a temporary joining device (process P7). The temporary joining device temporarily fastens the overlapping parts of the sheet S to each other in multiple places indicated by black dots in FIG. 3, for example, though this is not intended to be limiting. It will be noted that temporarily fastening the overlapping parts of the sheet S to each other here means joining the overlapping parts of the sheet S to each other to an extent that the wearer or a caregiver helping the wearer put on the diaper 1 can easily unjoin the overlapping parts by applying force. The reason that the overlapping parts of the sheets S are weakly joined to each other in this way is to allow the wearer, when putting on the diaper 1, to unjoin them at the temporarily joined places, unfold the parts that were folded by the sheet folding device, and use the diaper 1.

It will be noted that this temporary joining of the overlapping parts of the sheet S to each other is performed to inhibit the occurrence of problems such as the folded parts of the sheet S becoming unfolded (returning to their original state) and fluttering during conveyance such that the sheet S gets caught on some member or is conveyed off the conveyance path.

To temporarily join the overlapping parts of the sheet S to each other, an adhesive may be used or a joining method such as welding or ultrasonic welding may be used.

Next, the sheet S, which is conveyed in the second direction D2 in a state in which it has been folded by the sheet folding device, is cut by a first cutting device along the width direction W of the sheet S (process P8). Describing this now using FIG. 4, the first cutting device cuts the sheet S at the positions indicated by the dashed lines to form sheet pieces Sp. The formed sheet pieces Sp are further conveyed by the conveyance device in the second direction D2 (a direction orthogonal to the width direction W of the sheet pieces Sp (the sheet S)).

It will be noted that at the point in time when process P8 is implemented, the sheet S is not cut at the positions illustrated with the long dashed double-short dashed lines in FIG. 4. For that reason, each of the sheet pieces Sp includes the part 20 that becomes the front waist member 2 and the part 40 that becomes the back waist member 4 as shown in FIG. 4. It will be noted that the part 20 and the part 40 included in each sheet piece Sp become the front waist member 2 and the back waist member 4 of mutually different diapers 1.

Next, the sheet pieces Sp that are conveyed in the second direction D2 and are adjacent to each other in the second direction D2 are separated by a predetermined distance in the second direction D2 (process P9). Specifically, in process P9, the positions of the sheet pieces Sp are adjusted so that in the next process P10 the absorbent body 10 can be disposed so as to straddle the sheet pieces Sp that are adjacent to each other in the second direction D2 (so that the absorbent body 10 is disposed in an appropriate position relative to the part 40 of a first sheet piece Sp and the part 20 of a second sheet piece Sp trailing the first sheet piece Sp). Specifically, describing this now using FIG. 4, the first sheet piece Sp and the second sheet piece Sp are disposed apart from each other in a range in which the distance between the upstream end portion in the second direction D2 of the part 40 of the first sheet piece Sp located downstream in the second direction D2 and the downstream end portion in the second direction D2 of the part 20 of the second piece Sp trailing the first sheet piece Sp is shorter than the length of the absorbent body 10 in its lengthwise direction (the first direction D1). Furthermore, preferably the downstream end portion in the second direction D2 of the part 40 of the first sheet piece Sp located downstream in the second direction D2 and the upstream end portion of the part 20 in the second direction D2 of the second sheet piece Sp trailing the first sheet piece Sp are disposed apart from each other by a distance equal to or greater than the length of the absorbent body 10 in its lengthwise direction (the first direction D1).

Next, in process P10, the absorbent body 10, which is conveyed by an absorbent body conveyance device in the first direction D1 (the lengthwise direction of the absorbent body 10), is disposed on the skin surface side of the sheet pieces Sp so as to straddle the first sheet piece Sp that is one of the sheet pieces Sp and the second sheet piece Sp that is the sheet piece Sp adjacent to the first sheet piece Sp in the second direction D2.

It will be noted that at this time preferably the end portion of the first sheet piece Sp on the distal side relative to the second sheet piece Sp in the second direction D2 and the absorbent body 10 are disposed apart from each other in the second direction D2. Furthermore, preferably, the end portion of the second sheet piece Sp on the distal side relative to the first sheet piece Sp in the second direction D2 and the absorbent body 10 are disposed apart from each other in the second direction D2. Describing this now using FIG. 4, the downstream end portion in the second direction D2 of the part 40 of the first sheet piece Sp located downstream in the second direction D2 and the downstream end portion of the absorbent body 10 in the second direction D2 are disposed apart from each other, and the upstream end portion in the second direction D2 of the part 20 of the second sheet piece Sp trailing the first sheet piece Sp and the upstream end portion of the absorbent body 10 in the second direction D2 are disposed apart from each other.

Additionally, in process P10, an absorbent body joining device fixes the pair of sheet pieces Sp and the absorbent body 10 to each other. The fixing method at this time may be adhesion or may be thermal welding or ultrasonic welding for example.

Next, in process P11, the parts 20 and the parts 40 of the sheet pieces Sp to which the absorbent bodies 10 have been fixed are cut along the width direction W of the sheet pieces Sp and separated by a second cutting device. Describing this now with reference to FIG. 4, in process P11 the second cutting device cuts the sheet pieces Sp along the width direction W at the positions of the dashed lines. The diaper 1 is formed as a result of process P11 being performed.

It will be noted that the manufacturing process described here is merely an example and can be modified as appropriate.

For example, the manufacturing process of the diaper 1 may include processes other than those described here (e.g., a process of further bonding a sheet other than the first sheets A to the sheet S).

Furthermore, in the manufacturing process of the diaper 1, some of the processes described above (e.g., process P4 of bonding the first sheets A) may be omitted provided that doing so does not contradict the spirit of this disclosure.

Furthermore, in the manufacturing process of the diaper 1, some of the processes described may be replaced with other processes. For example, in the case of forming the loop structure of the hook-and-loop fastener on the sheet S by performing a process on the sheet S rather than by attaching the loop tape 8 to the sheet S, the process of attaching the loop tape 8 may be replaced with a process of forming the loop structure.

Furthermore, in the manufacturing process of the diaper 1, the order of the processes may be changed where not contradictory. For example, the order of process P2 and process P3 described above may be reversed.

Furthermore, in the manufacturing process of the diaper 1, the processes may be changed where not contradictory. For example, in the manufacturing process of the diaper 1 described above, the sheet pieces Sp are formed (process P8), and thereafter the sheet pieces Sp are cut in the width direction W to form the front waist member 2 and the back waist member 4 (process P11). However, as long as it is possible from a manufacturing standpoint, the front waist member 2 and the back waist member 4 may be formed as sheet pieces directly from the sheet S (without involving the process of forming the sheet pieces Sp), and the absorbent body 10 may be fixed to the sheet pieces.

### (3) Reducing Amount of Material in Wearable Article

In the diaper 1 that is a wearable article of this embodiment, the manufacturing method described above reduces the amount of material by bonding the first sheets A to only parts of the sheet S, disposing the first end E1 of the absorbent body 10 and the upper end portion of the front waist member 2 apart from each other, and disposing the second end E2 of the absorbent body 10 and the upper end portion of the back waist member 4 apart from each other.

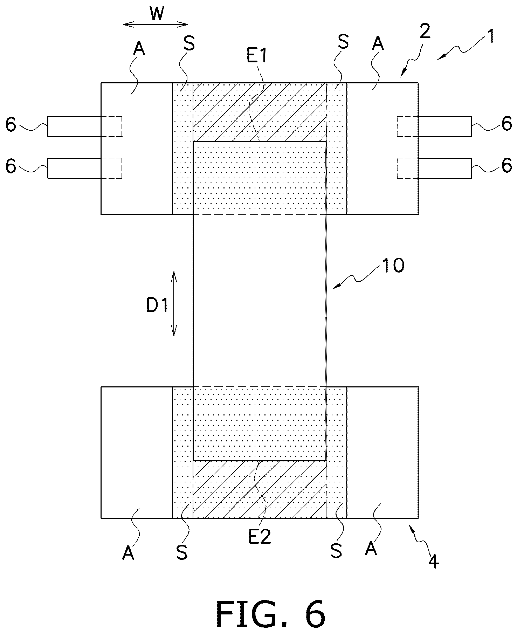

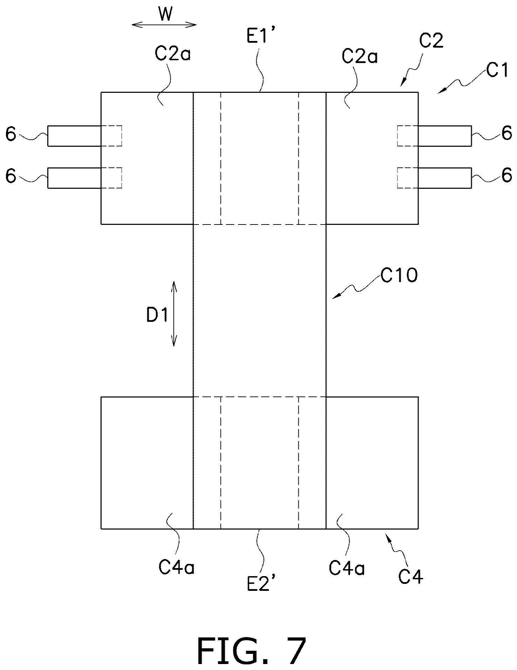

The reduction in the amount of material in the diaper 1 will now be described with reference to FIG. 6 and FIG. 7. FIG. 6 is a drawing showing the diaper 1 as viewed from the skin surface side and schematically shows places where material is reduced in the diaper 1. FIG. 7 is a drawing showing a conventional diaper C1 as viewed from the skin surface side and schematically shows the usage of material in the conventional diaper C1.

First, the conventional diaper C1 will be described.

A front waist member C2 of the conventional diaper C1, which corresponds to the front waist member 2 of this application, is not a sheet-like member extending continuously in the width direction W like the front waist member 2 but includes a pair of sheet pieces C2a that are disposed apart from each other in the width direction W (see FIG. 7). In FIG. 7, the end portions of the sheet pieces C2a on the center side of the front waist member C2 in the width direction W are illustrated with dashed lines. It will be noted that a sheet piece C2a is not disposed between the end portions of the pair of sheet pieces C2a illustrated with the dashed lines in FIG. 7 in the width direction W. Each sheet piece C2a is a sheet obtained by layering the sheet S and the first sheets A of this application.

Furthermore, a back waist member C4 of the conventional diaper C1, which corresponds to the back waist member 4 of this application, is not a sheet-like member extending continuously in the width direction W like the back waist member 4 but includes a pair of sheet pieces C4a that are disposed apart from each other in the width direction W (see FIG. 7). In FIG. 7, the end portions of the sheet pieces C4a on the center side of the back waist member C4 in the width direction W are illustrated with dashed lines. It will be noted that a sheet piece C4a is not disposed between the end portions of the pair of sheet pieces C4a illustrated with dashed lines in FIG. 7 in the width direction W. Each sheet piece C4a is a sheet obtained by layering the sheet S and the first sheets A of this application.

In contrast, in the diaper 1 of this disclosure, the front waist member 2 is a layered sheet in which the sheets A are bonded to both end portions in the width direction W of the sheet S that extends continuously in the width direction W. Furthermore, in the diaper 1 of this disclosure, the back waist member 4 is a layered sheet in which the sheets A are bonded to both end portions in the width direction W of the sheet S that extends continuously in the width direction W. Additionally, in the diaper 1 of this disclosure, the first end E1 of the absorbent body 10 and the upper end portion of the front waist member 2 are disposed apart from each other, and the second end E2 of the absorbent body 10 and the upper end portion of the back waist member 4 are disposed apart from each other.

In a case where the diaper 1 and the diaper C1 are the same size, as will be understood by comparing FIG. 6 and FIG. 7, in the diaper 1 of this disclosure, the length in the first direction D1 of the absorbent body 10 configured from ground pulp, a superabsorbent polymer, and nonwoven fabric can be shortened compared with the length in the first direction D1 of the absorbent body C10 of the conventional diaper C1, and the amount of material can be reduced (see the parts hatched with diagonal lines in FIG. 6).

It will be noted that although in the diaper 1 the length of the absorbent body 10 in the first direction D1 is shorter than the length of the absorbent body C10 of the conventional diaper C1, the width direction W length of the sheet S used in the front waist member 2 and the back waist member 4 is longer than that of the sheet S used in the front waist member C2 and the back waist member C4 of the conventional diaper C1 (because the sheet S spans the entire length of the front waist member 2 and the back waist member 4 in the width direction W). In other words, the amount of the sheet S used in the front waist member 2 and the back waist member 4 is greater than the amount of the sheet S used in the front waist member C2 and the back waist member C4. However, in the diaper 1, the first sheets A are not disposed in the parts of the front waist member 2 and the back waist member 4 that are hatched with dots, so the first sheets A can be reduced more than in the front waist member C2 and the back waist member C4 of the conventional diaper C1.

Consequently, in the diaper 1, it becomes possible to reduce the amount of material used for the front waist member 2 and the back waist member 4 compared with the amount of material used for the front waist member C2 and the back waist member C4. Furthermore, even if by design the amount of material used for the front waist member 2 and the back waist member 4 of the diaper 1 were to increase compared with the amount of material used for the front waist member C2 and the back waist member C4, the amount of the increase can be curbed.

### (4) Characteristics

(4-1)
The wearable article manufacturing method of the above embodiment is a method of manufacturing the diaper 1 serving as an example of a wearable article having the absorbent body 10, the front waist member 2 serving as an example of a first waist member, and the back waist member 4 serving as an example of a second waist member. The absorbent body 10 is disposed in the crotch of a wearer and extends in the first direction D1 between the first end E1 and the second end E2. The front waist member 2 is connected to the absorbent body 10 on the first end E1 side and is disposed on the front waist of the wearer when the diaper 1 is worn. The back waist member 4 is connected to the absorbent body 10 on the second end E2 side and is disposed on the back waist of the wearer when the diaper 1 is worn. The diaper 1 manufacturing method includes the process (process P1) of conveying in the second direction D2 the sheet S that is to be formed into the front waist member 2 and the back waist member 4 and the folding process (process P6). The second direction D2 is a direction orthogonal to the width direction W of the sheet S, and the width direction W of the sheet S is a direction along the waist of the wearer when the diaper 1 is worn by the wearer. In the folding process, both ends in the width direction W of the conveyed sheet S are folded toward the center C side of the sheet S in the width direction W.

In this diaper 1 manufacturing method, rather than a first sheet for forming the front waist member 2 and a second sheet for forming the back waist member 4 being conveyed parallel to each other with a gap between them, the sheet S for forming the front waist member 2 and the back waist member 4 is conveyed in a direction orthogonal to the width direction W and is further conveyed after being folded in the width direction W. For that reason, in this manufacturing method, at least manufacturing equipment downstream of the sheet S folding process can be downsized.

(4-2)
The method of manufacturing the diaper 1 of the above embodiment includes the process (process P10) of conveying the absorbent body 10, the process (process P8) of forming the sheet pieces Sp, the separation process (process P9), and the process (process P10) of fixing the sheet pieces Sp and the absorbent body 10 to each other. In the process of conveying the absorbent body 10, the absorbent body 10 is conveyed along the first direction D1. The process of forming the sheet pieces Sp is executed after the sheet S has been folded in the folding process (process P6). The process of forming the sheet pieces Sp is a process of forming the sheet pieces Sp by cutting the sheet S along the width direction W and conveying the formed sheet pieces Sp further in the second direction D2 (the width direction W of the sheet S). In the separation process, the sheet pieces Sp that are conveyed in the second direction D2 and are adjacent to each other in the second direction D2 are separated by a predetermined distance in the second direction D2. The process of fixing the sheet pieces Sp and the absorbent body 10 to each other is executed after the separation process (process P9). In the process of fixing the sheet pieces Sp and the absorbent body 10 to each other, the absorbent body 10 conveyed along the first direction D1 is disposed so that it straddles the first sheet piece Sp that is one of the sheet pieces Sp and the second sheet piece Sp that is the sheet piece Sp adjacent to the first sheet piece Sp in the second direction D2, and the sheet pieces Sp and the absorbent body 10 are fixed to each other.

In the diaper 1 manufacturing method of this embodiment, the absorbent body 10 is conveyed along the first direction D1, which is the lengthwise direction of the absorbent body 10, and is combined with the sheet S (sheet pieces Sp) that has been folded in the width direction W. Because the manufacturing method is configured in this way, manufacturing equipment in the width direction orthogonal to the conveyance direction of the absorbent body 10 and the sheet S (sheet pieces Sp) can be downsized.

(4-3)
In the method of manufacturing the diaper 1 of the above embodiment, the length L of the sheet S in the width direction W after the sheet S has been folded by the folding process (process P6) is from 1.0 times to 1.3 times the width B of the absorbent body 10 in the third direction D3. More preferably, the length L of the sheet S in the width direction W after the sheet S has been folded by the folding process (process P6) is from 1.05 times to 1.2 times the width B of the absorbent body 10 in the third direction D3. The third direction D3 of the absorbent body 10 is a direction orthogonal to the first direction D1 of the absorbent body 10 (the lengthwise direction of the absorbent body 10).

In the method of manufacturing the diaper 1 of this embodiment, the sheet S is folded such that it becomes, in the width direction W thereof, at least 1.3 times or less the width B of the absorbent body 10 and is thereafter conveyed in the second direction D2 orthogonal to the width direction W. Therefore, the size of the manufacturing equipment in a direction orthogonal to the conveyance direction of the sheet S can be reduced.

(4-4)
The method of manufacturing the diaper 1 of the above embodiment includes the process (process P2) of attaching the hook tapes 6 serving as an example of tape-like members to the sheet S and the process (process P5) of folding the hook tapes 6. In the process of fixing the hook tapes 6 to the sheet S, the hook tapes 6 that fix the front waist member 2 and the back waist member 4 to each other when the diaper 1 has been formed are attached to both end portions in the width direction W of the sheet S before the sheet S is folded by the folding process (process P6). In the process of folding the hook tapes 6, the hook tapes 6 are folded toward the center C side of the sheet S in the width direction W before the sheet S is folded in the folding process.

In the diaper 1 manufacturing method of this embodiment, the occurrence of a situation where the width of the conveyance path of the sheet S increases due to the presence of the hook tapes 6 can be inhibited.

(4-5)
The method of manufacturing the diaper 1 of the above embodiment includes the process (process P7) of temporarily fastening the overlapping parts of the sheet S to each other, which is executed after the sheet S has been folded in the folding process (process P6).

In method of manufacturing the diaper 1 of this embodiment, the occurrence of problems such as the folded parts of the sheet S becoming unfolded and fluttering during conveyance such that the sheet S gets caught on some member or the sheet S is conveyed off the conveyance path can be inhibited.

(4-6)
The method of manufacturing the diaper 1 of the above embodiment includes the process (process P4) of bonding the first sheets A serving as auxiliary sheets along the second direction D2 to both end portions, in the width direction, of the sheet S conveyed in the second direction D2 before the sheet S is folded in the folding process (process P6). In the process of bonding the first sheets A, the first sheet A that is bonded to one end portion in the width direction W and the first sheet A that is bonded to the other end portion in the width direction W are disposed apart from each other in the width direction W. In the process of fixing the sheet pieces Sp and the absorbent body 10 to each other, the end portion of the first sheet piece Sp on the distal side relative to the second sheet piece Sp (which is the sheet piece Sp adjacent to the first sheet piece Sp in the second direction D2) in the second direction D2 and the absorbent body 10 are disposed apart from each other in the second direction D2. Furthermore, in the process of fixing the sheet pieces Sp and the absorbent body 10 to each other, the end portion of the second sheet piece Sp on the distal side relative to the first sheet piece Sp in the second direction D2 and the absorbent body 10 are disposed apart from each other in the second direction D2.

In the method of manufacturing the diaper 1 of this embodiment, the amount of material that becomes used to manufacture the diaper 1 can be curbed.

### <Additional Notes>

Finally, the technical idea that can be grasped from the above embodiment is appended below.

A method of manufacturing a wearable article of a first aspect is a method of manufacturing a wearable article having an absorbent body, a first waist member, and a second waist member. The absorbent body is disposed in the crotch of a wearer and extends in a first direction between a first end and a second end. The first waist member is connected to the absorbent body on the first end side and is disposed on the front waist of the wearer when the wearable article is worn. The second waist member is connected to the absorbent body on the second end side and is disposed on the back waist of the wearer when the wearable article is worn. The method of manufacturing the wearable article includes a process of conveying in a second direction a sheet that is to be formed into the first waist member and the second waist member and a folding process. The second direction is a direction orthogonal to the width direction of the sheet, and the width direction of the sheet is a direction along the waist of the wearer when the wearable article is worn by the wearer. In the folding process, both ends in the width direction of the conveyed sheet are folded toward a center side of the sheet in the width direction.

In the wearable article manufacturing method of the first aspect, rather than a first sheet for forming the first waist member and a second sheet for forming the second waist member being conveyed parallel to each other with a gap between them, the sheet for forming the first waist member and the second waist member is conveyed in a direction orthogonal to the width direction and is further conveyed after being folded in the width direction. For that reason, in the wearable article manufacturing method of the first aspect, at least manufacturing equipment downstream of the sheet folding process can be downsized.

A method of manufacturing the wearable article of a second aspect is the method of manufacturing the wearable article of the first aspect, further including a process of conveying the absorbent body, a process of forming sheet pieces, a separation process, and a process of fixing the sheet pieces and the absorbent body to each other. In the process of conveying the absorbent body, the absorbent body is conveyed along the first direction. The process of forming the sheet pieces is executed after the sheet has been folded in the folding process. The process of forming the sheet pieces is a process of forming the sheet pieces by cutting the sheet along the width direction and conveying the formed sheet pieces further in the second direction (the width direction of the sheet). In the separation process, the sheet pieces that are conveyed in the second direction and are adjacent to each other in the second direction are separated by a predetermined distance in the second direction. The process of fixing the sheet pieces and the absorbent body to each other is executed after the separation process. In the process of fixing the sheet pieces and the absorbent body to each other, the absorbent body conveyed along the first direction is disposed so that it straddles a first sheet piece that is one of the sheet pieces and a second sheet piece that is the sheet piece adjacent to the first sheet piece in the second direction, and the sheet pieces and the absorbent body are fixed to each other.

In the wearable article manufacturing method of the second aspect, the absorbent body is conveyed along the first direction, which is the lengthwise direction of the absorbent body, and is combined with the sheet (sheet pieces) that has been folded in the width direction. Because the manufacturing method is configured in this way, manufacturing equipment in a width direction orthogonal to the conveyance direction of the absorbent body and the sheet (sheet pieces) can be downsized.

A method of manufacturing the wearable article of a third aspect is the method of manufacturing the wearable article of the second aspect, wherein the length of the sheet in the width direction after the sheet has been folded by the folding process is from 1.0 times to 1.3 times the width of the absorbent body in a third direction. The third direction of the absorbent body is a direction orthogonal to the first direction of the adsorbent body.

In the wearable article manufacturing method of the third aspect, the sheet is folded such that it becomes, in the width direction thereof, at least 1.3 times or less the width (the length in the third direction) of the absorbent body and is thereafter conveyed in a direction orthogonal to the width direction. Therefore, the size of the manufacturing equipment in a direction orthogonal to the conveyance direction of the sheet can be reduced.

A method of manufacturing the wearable article of a fourth aspect is the method of manufacturing the wearable article of any of the first aspect to the third aspect, further including a process of attaching tape-like members to the sheet and a process of folding the tape-like members. In the process of fixing the tape-like members to the sheet, the tape-like members that fix the first waist member and the second waist member to each other when the wearable article has been formed are attached to both end portions in the width direction of the sheet before the sheet is folded by the folding process. In the process of folding the tape-like members, the tape-like members are folded toward a center side of the sheet in the width direction before the sheet is folded in the folding process.

In the wearable article manufacturing method of the fourth aspect, the occurrence of a situation where the width of the conveyance path of the sheet increases due to the presence of the tape-like members can be inhibited.

A method of manufacturing the wearable article of a fifth aspect is the method of manufacturing the wearable article of any of the first aspect to the fourth aspect, further including a process of temporarily fastening overlapping parts of the sheet to each other, which is executed after the sheet has been folded in the folding process.

In the wearable article manufacturing method of the fifth aspect, the occurrence of problems such as the folded parts of the sheet becoming unfolded and fluttering during conveyance such that the sheet gets caught on some member or the sheet is conveyed off the conveyance path can be inhibited.

A method of manufacturing the wearable article of a sixth aspect is the method of manufacturing the wearable article of the second aspect, further including a process of bonding auxiliary sheets along the second direction to both end portions, in the width direction, of the sheet conveyed in the second direction before the sheet is folded in the folding process. In the process of bonding the auxiliary sheets, the auxiliary sheet that is bonded to one end portion in the width direction and the auxiliary sheet that is bonded to the other end portion in the width direction are disposed apart from each other in the width direction. In the process of fixing the sheet pieces and the absorbent body to each other, an end portion of the first sheet piece on a distal side from the second sheet piece in the second direction and the absorbent body are disposed apart from each other in the second direction. Furthermore, in the process of fixing the sheet pieces and the absorbent body to each other, an end portion of the second sheet piece on a distal side from the first sheet piece in the second direction and the absorbent body are disposed apart from each other in the second direction.

In the wearable article manufacturing method of the sixth aspect, the amount of material that is used to manufacture the wearable article can be curbed.

### REFERENCE SIGNS LIST

1 Diaper (Wearable Article)
- 2: Front Waist Member (First Waist Member)
- 4: Back Waist Member (Second Waist Member)
- 6: Hook Tapes (Tape-like Members)
- 10: Absorbent Body
- A: First Direction (Auxiliary Sheets)
- B: Width of Absorbent Body in Third Direction
- D1: First Direction
- D2: Second Direction
- D3: Third Direction
- E1: First End
- E2: Second End
- L: Length of Sheet in Width Direction after Sheet has been Folded by Folding Process
- S: Sheet
- Sp: Sheet Pieces
- W: Width Direction

## Claims

1. A method of manufacturing a wearable article having an absorbent body that is disposed in the crotch of a wearer and extends in a first direction between a first end and a second end, a first waist member that is connected to the absorbent body on a side of the first end and is disposed on a front waist of the wearer when the wearable article is worn, and a second waist member that is connected to the absorbent body on a side of the second end side and is disposed on a back waist of the wearer when the wearable article is worn, the manufacturing method comprising:
a process of conveying a sheet, which is to be formed into the first waist member and the second waist member and whose width direction coincides with a direction along the waist of the wearer when the wearable article is worn by the wearer, in a second direction orthogonal to the width direction; and
a folding process of folding both ends in the width direction of the conveyed sheet toward a center side of the sheet in the width direction.

2. The method of manufacturing the wearable article according to claim 1, further comprising
a process of conveying the absorbent body along the first direction,
a process of forming sheet pieces by cutting the sheet along the width direction after the sheet has been folded in the folding process and conveying the sheet pieces further in the second direction,
a separation process of separating, by a predetermined distance in the second direction, the sheet pieces that are conveyed in the second direction and are adjacent to each other in the second direction, and
a process, which is executed after the separation process, of disposing the absorbent body conveyed along the first direction so that it straddles a first sheet piece that is one of the sheet pieces and a second sheet piece that is the sheet piece adjacent to the first sheet piece in the second direction and fixing the sheet pieces and the absorbent body to each other.

3. The method of manufacturing the wearable article according to claim 2, wherein the length of the sheet in the width direction after the sheet has been folded by the folding process is from 1.0 times to 1.3 times the width of the absorbent body in a third direction orthogonal to the first direction.

4. The method of manufacturing the wearable article according to any one of claims 1 to 3, further comprising
a process of attaching, to both end portions in the width direction of the sheet before the sheet is folded by the folding process, tape-like members that fix the first waist member and the second waist member to each other when the wearable article has been formed, and
a process of folding the tape-like members toward a center side of the sheet in the width direction before the sheet is folded by the folding process.

5. The method of manufacturing the wearable article according to any one of claims 1 to 4, further comprising a process of temporarily fastening overlapping parts of the sheet to each other after the sheet has been folded in the folding process.

6. The method of manufacturing the wearable article according to claim 2, further comprising a process of bonding auxiliary sheets along the second direction to both end portions, in the width direction, of the sheet conveyed in the second direction before the sheet is folded in the folding process,
wherein
in the process of bonding the auxiliary sheets, the auxiliary sheet that is bonded to one end portion in the width direction and the auxiliary sheet that is bonded to the other end portion in the width direction are disposed apart from each other in the width direction, and
in the process of fixing the sheet pieces and the absorbent body to each other, an end portion of the first sheet piece on a distal side relative to the second sheet piece in the second direction and the absorbent body are disposed apart from each other in the second direction, and an end portion of the second sheet piece on a distal side relative to the first sheet piece in the second direction and the absorbent body are disposed apart from each other in the second direction.
